# EUROPEAN PATENT APPLICATION

(11) **EP 4 349 292 A2**
(43) Date of publication of application: **10.04.2024**
(21) Application number: 24158977.9
(22) Date of filing: 07.09.2020
(51) Int. Cl.: A61B 18/20

(54) **SELECTIVE LASER STIMULATION OF CORNEAL STEM CELLS**

(30) Priority: 12.09.2019 US 201962899162 P
(62) Divisional of application: 20864109.2
(71) Applicant: Belkin Vision Ltd., 8122214 Yavne (IL)
(72) Inventor: Belkin, Michael, 5404402 Givat Shmu'el (IL); Sacks, Zachary, 7173817 Modiin (IL)
(74) Representative: Beck Greener LLP

(57) **Abstract**

Apparatus (20) for ophthalmic treatment includes an optical unit (30), which includes a camera (54), which is configured to capture an image of an eye (25) of a patient, a laser (48), which is configured to emit pulses of laser radiation, and beam conditioning and scanning optics (49, 50), which are configured to focus and direct the laser radiation to impinge on an anterior surface of the eye. A controller (44) is configured to analyze the image so as to identify a limbus of the eye, and to control the optical unit so that the laser radiation impinges on a set of one or more locations on the anterior surface of the eye that are in a vicinity of the limbus with a pulse duration and an energy selected so as to stem cells at the one or more locations.

## Description

### FIELD OF THE INVENTION

The present invention relates generally to methods and apparatus for treatment of the eye, and particularly to treating conditions of the cornea.

### BACKGROUND

The corneal epithelium consists of a few layers of cells on the anterior surface of the eye. These cells are almost continuously exposed and thus are constantly replaced. The cornea is the most highly innervated tissue in the body. Consequently, any disturbance of its epithelial surface leads to symptoms such as pain and discomfort, which may be severe. Furthermore, the corneal epithelium and its tear layer coverage are responsible for the proper optical transparency of the eye.

Dry eye syndrome occurs when either the eye does not produce enough tears or when the tears evaporate too quickly. It affects 5-34% of people to some degree, and up to 70% among older people. Symptoms of dry eye include irritation, redness, discharge, and easily fatigued eyes. More serious cases can lead to blurred vision and even corneal scarring.

The corneal endothelium lines the inner surface of the cornea and is responsible for the transparency of the tissue. Defects in the endothelium, whether due to diseases or trauma, can lead to corneal swelling and visual impairment.

Light can act on different mechanisms within cellular tissue to stimulate or suppress biological activity, in a process commonly referred to as photobiomodulation (PBM) or low light level therapy (LLLT). Therapy of this sort has been proposed for treatment of various conditions of the eye, although its clinical efficacy has not been conclusively established. For example, U.S. Patent Application Publication 2016/0067087 describes a wearable ophthalmic phototherapy device and associated treatment methods to expose an eye to selected multi-wavelengths of light to promote the healing of damaged or diseased eye tissue. The device includes a frame having a front piece and two earpieces extending from the front piece; and at least one light source producing a light beam having a therapeutic wavelength and disposed within or on the frame.

As another example, U.S. Patent 9,192,780 describes a system and method for treatment of cells and, in particular, visual pathway disorders using photomodulation and/or photorejuvenation, particularly of retinal epithelial cells. The process of treating retinal cells to reduce or reverse the effects of visual pathway disorders employs a narrowband source of multichromatic light applied to the retinal cells to deliver a very low energy fluence.

### SUMMARY

Embodiments of the present invention that are described hereinbelow provide improved apparatus and methods for treatment of the eye.

There is therefore provided, in accordance with an embodiment of the invention, apparatus for ophthalmic treatment, including an optical unit, which includes a camera, which is configured to capture an image of an eye of a patient, a laser, which is configured to emit pulses of laser radiation, and beam conditioning and scanning optics, which are configured to focus and direct the laser radiation to impinge on an anterior surface of the eye. A controller is configured to analyze the image so as to identify a limbus of the eye, and to control the optical unit so that the laser radiation impinges on a set of one or more locations on the anterior surface of the eye that are in a vicinity of the limbus with a pulse duration that is less than 1 µs and an energy that is less than 5 mJ per pulse, whereby the laser radiation stimulates stem cells at the one or more locations.

In a disclosed embodiment, the laser radiation is focused to impinge on each of the one or more locations with a beam diameter no greater than 1 mm. Additionally or alternatively, the pulse duration is less than 100 ns and may be less than 10 ns or even less than 5 ns. Further additionally or alternatively, the energy per pulse is less than 3 mJ. Typically, the laser radiation is focused to impinge on each of the one or more locations with a fluence less than 0.4 J/cm².

In some embodiments, the controller is configured to actuate the laser so as to apply multiple pulses to each of the one or more locations. In a disclosed embodiment, the multiple pulses include no more than 200 pulses at each location, and possibly no more than 100 pulses at each location. Additionally or alternatively, the multiple pulses deliver the laser radiation with a cumulative energy that is no greater than 100 mJ at each location and may be no greater than 70 mJ or even no greater than 40 mJ.

In a disclosed embodiment, the one or more locations on the anterior surface of the eye are displaced by at least 1 mm from the limbus and possibly by at least 2 mm from the limbus.

Additionally or alternatively, the beam conditioning and scanning optics are configured to direct the laser radiation to impinge on the anterior surface of the eye at an oblique angle relative to a direction of an optical axis of the eye. In one embodiment, the beam conditioning and scanning optics include a ring mirror, which is positioned around the optical axis of the eye so as to reflect the beam toward multiple locations in the vicinity of the limbus at oblique angles.

There is also provided, in accordance with an embodiment of the invention, apparatus for ophthalmic treatment, including an optical unit as described above. A controller is configured to analyze the image so as to identify a limbus of the eye, and to control the optical unit so that the laser radiation impinges on a set of one or more locations on the anterior surface of the eye in a vicinity of the limbus with a pulse duration that is between 1 ms and 500 ms and an energy that is less than 100 mJ per pulse, whereby the laser radiation stimulates stem cells at the one or more locations.

In some embodiments, the pulse duration is less than 50 ms and may be less than 10 ms or even less than 5 ms. Additionally or alternatively, the energy per pulse is less than 50 mJ and may be less than 5 mJ. Typically, the laser radiation is focused to impinge on each of the one or more locations with a fluence less than 30 J/cm².

In some embodiments, the controller is configured to actuate the laser so as to apply multiple pulses to each of the one or more locations. In a disclosed embodiment, the multiple pulses include no more than no more than 2000 pulses at each location and possibly no more than 500 pulses at each location or even no more than 200 pulses at each location. Additionally or alternatively, the multiple pulses deliver the laser radiation with a cumulative energy that is no greater than 10 J at each location and possibly no greater than 1 J or even no greater than 400 mJ.

There is additionally provided, in accordance with an embodiment of the invention, a method for ophthalmic treatment, which includes identifying a limbus of an eye of a patient, and directing pulses of laser radiation to impinge on a set of one or more locations on an anterior surface of the eye in a vicinity of the limbus with a pulse duration that is less than 1 µs and an energy that is less than 5 mJ per pulse, so as to stimulate stem cells at the one or more locations.

There is further provided, in accordance with an embodiment of the invention, a method for ophthalmic treatment, which includes identifying a limbus of an eye of a patient, and directing pulses of laser radiation to impinge on a set of one or more locations on an anterior surface of the eye in a vicinity of the limbus with a pulse duration that is less than 100 ms and an energy that is less than 100 mJ per pulse, so as to stimulate stem cells at the one or more locations.

There is moreover provided, in accordance with an embodiment of the invention, apparatus for treating an eye of a patient. The apparatus includes an optical unit, including a laser, which is configured to emit laser radiation, one or more mirrors positioned around an optical axis of the eye, and beam conditioning and scanning optics, which are configured to focus and direct the laser radiation to reflect from the one or more mirrors so as to impinge on an anterior surface of the eye at an oblique angle relative to the optical axis. A controller is configured to control the optical unit so that the laser radiation impinges on the eye at the oblique angle in a set of one or more locations in a vicinity of the limbus with a fluence selected so as to stimulate stem cells at the one or more locations.

In a disclosed embodiment, the one or more mirrors include a ring mirror, which surrounds the optical axis.

In one embodiment, the controller is configured to control the optical unit so that the laser radiation impinges on and stimulates the stem cells in an epithelium of the eye. Alternatively or additionally, the controller is configured to control the optical unit so that the laser radiation impinges on and stimulates the stem cells in a stroma of the eye. Further additionally or alternatively, the controller is configured to control the optical unit so that the laser radiation impinges on and stimulates the stem cells in an endothelium of the eye.

There is furthermore provided, in accordance with an embodiment of the invention, a method for ophthalmic treatment, which includes placing a gonioscope in contact with a cornea of an eye of a patient. Pulses of laser radiation are directed through the gonioscope to reflect from a side mirror surface of the gonioscope and then to pass obliquely through the cornea and impinge on endothelial tissue in a vicinity of a trabecular meshwork of the eye with a pulse energy and duration selected so as to stimulate stem cells in the endothelial tissue.

In some embodiments, the pulses have a pulse duration that is less than 1 µs and an energy that is less than 0.1 mJ per pulse. In a disclosed embodiment, directing the pulses includes controlling the laser radiation to impinge on the endothelial tissue with a fluence no greater than 0.1 J/cm². Additionally or alternatively, the pulses have a pulse duration that is between 1 and 500 ms and a fluence no greater than 10 J/cm².

The present invention will be more fully understood from the following detailed description of the embodiments thereof, taken together with the drawings in which:

### BRIEF DESCRIPTION OF THE DRAWINGS

Fig. 1 is schematic side view of a system for ophthalmic treatment, in accordance with an embodiment of the invention;
Fig. 2 is a block diagram that schematically shows details of a laser treatment device in the system of Fig. 1, in accordance with an embodiment of the invention;
Fig. 3 is a schematic sectional illustration of structures in an eye under treatment by laser radiation, in accordance with an embodiment of the invention;
Fig. 4 is a schematic sectional idealized illustration of a limbal area of an eye showing propagation of a laser beam through the area, in accordance with an embodiment of the invention;
Figs. 5A and 5B are schematic idealized sectional illustrations of tissue in an eye showing distributions of optical fluence under irradiation by laser beams of different focal properties, in accordance with an embodiment of the invention; .
Figs. 6 and 7 are schematic sectional views of systems for ophthalmic treatment, in accordance with alternative embodiments of the invention; and
Fig. 8 is a schematic sectional illustration of apparatus for ophthalmic treatment, in accordance with yet another embodiment of the invention.

### DETAILED DESCRIPTION OF EMBODIMENTS

### OVERVIEW

Stem cells play important roles in homeostasis and repair of tissues in the eye. For example, inadequate stem cell activity appears to be one of the causes of dry eye and other pathologic conditions of the cornea. Stimulation of stem cell activity can be an effective treatment for such conditions.

LLLT using visible or infrared laser radiation has been found to stimulate stem cell activity in various tissues in the body. This sort of therapy typically uses CW laser radiation at moderate energy levels. Application of such radiation to the eye, however, can have serious unintended consequences, including transient and possibly permanent damage to the retina and other physiological structures. For example, laser irradiation of the region around the limbus with sufficient intensity has been shown to reduce intraocular pressure, as described in U.S. Patent 10,702,416, whose disclosure is incorporated herein by reference. This effect is helpful in treating glaucoma, but it can lead to dangerously low pressure if applied to patients whose initial intraocular pressure is in the normal range.

In response to these needs and constraints, embodiments of the present invention that are described herein provide apparatus and methods for treatment of the cornea using pulsed laser radiation, with pulse parameters chosen specifically to stimulate cornea stem cells while minimizing undesired collateral effects. The disclosed techniques can be adapted to stimulate the stem cells in any and all of the layers of the cornea, including the epithelium, stroma, and endothelium. They are particularly effective in stimulating epithelial stem cell activity and thus in treating conditions of the corneal epithelium, such as dry eye. In an alternative embodiment, corneal endothelial stem cells are activated by this sort of laser irradiation.

The embodiments that are described hereinbelow use an optical unit comprising a camera, which captures images of the eye of a patient. A laser in the optical unit emits pulses of laser radiation, which are focused and directed by beam conditioning and scanning optics to impinge on the anterior surface of the eye. A controller analyzes the image so as to identify the limbus of the eye, and then controls the optical unit so that the laser radiation impinges on a set of one or more locations on the anterior surface of the eye in the vicinity of the limbus, including locations that are displaced by a predefined radial distance of at least 1 mm from the limbus, and possibly by 2, 3 or even 4 mm. These are locations at which stem cell stimulation can be effective while avoiding undesired effects on underlying structures. Alternatively, the locations to be irradiated are identified by other methods of image analysis and eye tracking that are known in the art, for example pupil tracking or pattern recognition.

There are two regimes of laser pulse parameters that the inventors have found to be effective in stimulating corneal stem cells (particularly in the epithelium), while minimizing other undesired effects. In some embodiments, the laser radiation impinges on the eye with a pulse duration that is less than 1 µs and an energy that is less than 5 mJ per pulse. In alternative embodiments, the pulse duration can range up to 100 ms, with a pulse energy up to 100 mJ per pulse. The fluence of laser radiation on the eye in each pulse is held below a predefined limit, for example less than 2 J/cm² in the short pulse domain (less than 1 µs) or less than 120 J/cm² in the longer pulse domain (up to 100 ms). The cumulative energy applied to each location is similarly held below a limit that is chosen so as to provide substantial stem cell stimulation while avoiding collateral effects.

In either case, the laser beam is typically focused to a small spot at each of the locations, for example with a beam diameter of 1 mm or less. The use of a small spot size of this sort is advantageous particularly in concentrating the beam intensity, and hence the stimulation of stem cells, in the epithelial layer. The intensity reaching the endothelium, which can affect structures such as the trabecular meshwork, is much smaller.

Some embodiments of the present invention make use of the inherent scattering properties of the tissue in the limbus. Simulations have shown that only approximately 11% of the laser energy that is incident on the limbus reaches the underlying trabecular meshwork. A clinical study conducted by the inventors showed that irradiation of the trabecular meshwork by laser pulses having a pulse energy of 0.8 mJ on the outer surface of the limbal region was effective at reducing intraocular pressure. A dose response has been observed in this sort of irradiation, meaning that higher energies result in greater pressure reduction. Thus, in some embodiments of the present invention, laser pulse energies below 0.8 mJ are used so that only the corneal epithelial and stromal stem cells are stimulated. Further energy reduction can be useful in ensuring that endothelial stem cells are not stimulated, so as to avoid undesired reduction of the intraocular pressure. In alternative embodiments, however, the energy and direction of the incident laser beam are chosen so that the endothelial stem cells are significantly stimulated.

There appear to be multiple pathways by which laser radiation can stimulate stem cells in the eye, including, but not limited to, thermal heating, nonlinear absorption processes, and photo-acoustic processes. Nanosecond laser pulses require about two orders of magnitude less fluence than millisecond-range pulses to achieve similar levels of stem cell stimulation. At the levels of fluence used in embodiments of the present invention, the attenuation of the laser energy propagating through the limbus is independent of pulse duration and energy, such that longer laser pulses with higher energy will also be attenuated by approximately 11%.

### SYSTEM DESCRIPTION

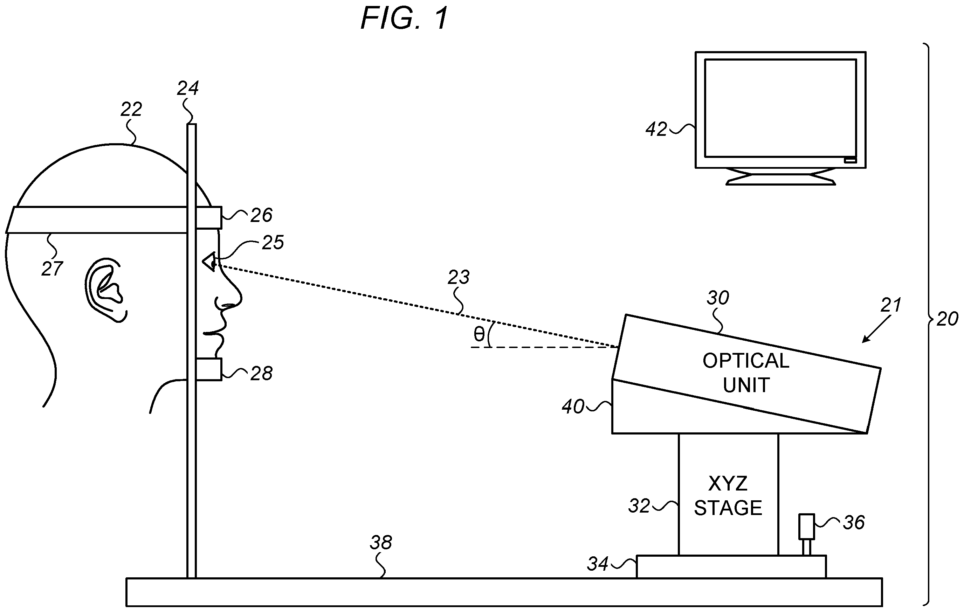

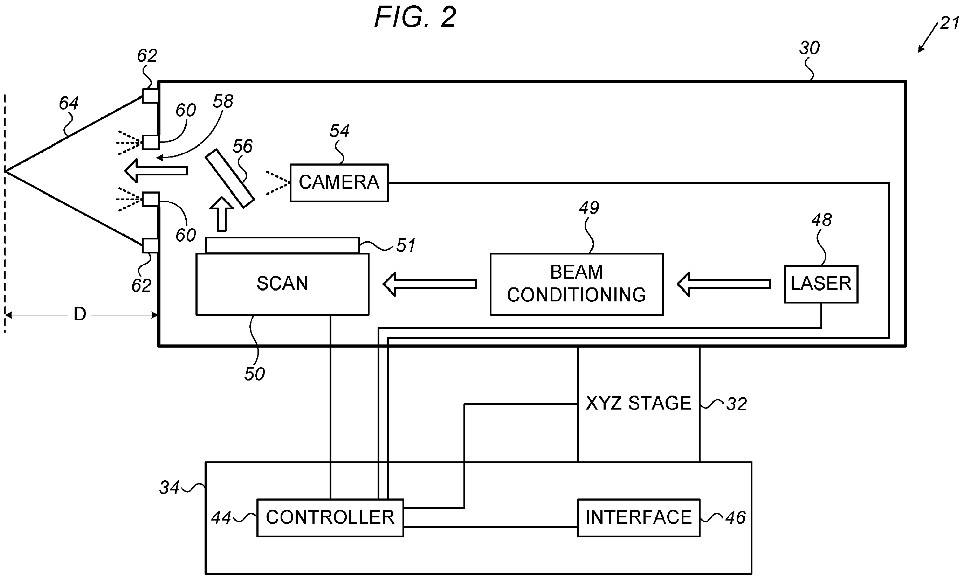

Reference is now made to Figs. 1 and 2, which schematically illustrate a system 20 for ophthalmic treatment using LLLT, in accordance with an embodiment of the invention. Fig. 1 is a side view of the system, while Fig. 2 is a block diagram showing details of a laser treatment device 21 in system 20. System 20 is similar in its design to a system for direct laser selective trabeculoplasty that is described in PCT International Publication WO 2020/008323, whose disclosure is incorporated herein by reference. In the present embodiment, however, the laser beam parameters and treatment modality in system 20 are specifically adapted for targeted stimulation of stem cells, while minimizing the effect of the laser beam on the trabecular meshwork.

Treatment device 21 comprises an optical unit 30, comprising a radiation source 48, such as a laser, which outputs a beam of pulsed radiation toward an eye 25 of a patient 22. Optical unit 30 further comprises beam-conditioning optics 49, for adjusting the focal properties of the laser beam, so that the beam is incident on the surface of eye 25 with the desired spot size and energy. A scanner 50 directs the beam toward the desired target location for the LLLT treatment. Scanner 50 may comprises, for example, one or more galvanometric mirrors or any other suitable type of optical scanner that is known in the art. An F-theta lens 51 can be used to preserve uniform beam properties over the range of the scan. A beam combiner 56 directs the laser beam through an aperture 58 at the front of optical unit 30 toward eye 25.

Optical unit 30 further comprises a camera 54, which acquires images of eye 25 via beam combiner 56 before and during the LLLT procedure. In the pictured embodiment, to aid in imaging of the eye, optical unit 30 comprises an illumination source 60 comprising, for example, one or more light emitting diodes (LEDs), such as a ring of LEDs surrounding aperture 58. A controller 44 processes the images output by camera 54 and, based on the images, controls radiation source 48, as well as optics 49 and scanner 50. Specifically, for purposes of the LLLT, the controller identifies the limbus of eye 25, and then aims the laser beam to direct pulses of radiation of the desired fluence toward locations in the vicinity of the limbus, meaning locations that are on the limbus or are displaced radially from the limbus by an appropriate distance along the anterior surface of the eye. The limbus can be identified using any suitable method of image processing that is known in the art, for example by finding the circular edge between the iris and sclera in the image. Methods for locating the limbus are described further in the above-mentioned U.S. Patent 10,702,416.

Typically, as a preliminary step before beginning treatment, the user of system 20, such as an ophthalmologist, positions optical unit 30 at a predefined distance D from the eye using a control mechanism 36, such as a joystick. To facilitate this positioning, optical unit 30 may comprise multiple beam emitters 62 (comprising, for example, laser diodes), which direct respective range-finding beams 64 toward the eye. Beams 64 form a composite pattern on the eye, which enables the user to ascertain that the optical unit is at the predefined distance.

Optical unit 30 is mounted on an XYZ stage 32, which is controlled by control mechanism 36. Using control mechanism 36, the user of system 20 positions the optical unit at the appropriate position prior to treating the eye of the patient. In some embodiments, XYZ stage 32 comprises one or more motors, and control mechanism 36 is connected to the XYZ stage via interface circuitry 46 and controller 44. In other embodiments, XYZ stage 32 is controlled manually by manipulating the control mechanism.

During operation, patient 22 places his head on a headrest 24, which is mounted on a horizontal surface 38, such as a tray or tabletop. Headrest 24 comprises a forehead rest 26 and a chinrest 28. During the LLLT procedure, patient 22 presses his forehead against forehead rest 26 while resting his chin on chinrest 28. An immobilization strap 27 secures the patient's head from behind and thus keep the patient's head pressed against the headrest.

In the pictured embodiment, a base unit 34 of device 21 is mounted on surface 38, and XYZ stage 32 is mounted on base unit 34. In such embodiments, controller 44 and interface circuitry 46 may be contained within the base unit. In other embodiments, the XYZ stage is mounted directly on surface 38.

It can be advantageous, as shown in Fig. 1, that while irradiating the patient's eye, optical unit 30 is directed obliquely upward toward the eye while the eye gazes obliquely downward toward the optical unit, i.e., an optical path 23 between the eye and the optical unit is oblique, rather than horizontal. For example, optical path 23 may be oriented at an angle θ of between five and twenty degrees, in order to reduce occlusion of the patient's eye by the patient's upper eyelid and associated anatomy. Optionally, for additional exposure of the eye, a finger, a speculum, or another tool may be used to retract one or both of the eyelids. In the embodiment shown in Fig. 1, the oblique orientation of optical path 23 is achieved by mounting optical unit 30 on a wedge 40, which is mounted on XYZ stage 32. Alternatively or additionally, the patient's head may be intentionally tilted in headrest 24.

System 20 optionally comprises a monitor 42, which displays the images of eye 25 that are acquired by camera 54 and enables the user to verify proper alignment and operation of the system. Monitor 42 can be connected directly to controller 44 over a wired or wireless communication interface or indirectly via an external processor, such as a processor belonging to a standard computer.

The configuration of system 20 and treatment device 21 that is shown in Figs. 1 and 2 is presented and described here by way of example only. The principles of LLLT treatment that are described herein may alternatively be implemented, *mutatis mutandis,* in other system configurations that are capable of irradiating the patient's eye at the appropriate locations with suitable beam parameters, as defined herein. All such alternative implementations are considered to be within the scope of the present invention.

Controller 44 typically comprises a general-purpose microprocessor, which is programmed in software to carry out the functions described herein and has appropriate interfaces for communicating with the other elements of system 20. The software may be downloaded to the controller in electronic form, over a network, for example. Alternatively or additionally, the software may be provided and/or stored on non-transitory tangible media, such as magnetic, optical, or electronic memory. Such software, when provided to the controller, produces a machine or special-purpose computer, configured to perform the tasks described herein. Alternatively or additionally, at least some of the functions of controller 44 may be implemented in dedicated or programmable hardware logic, for example using one or more Application-Specific Integrated Circuits (ASICs) or Field-Programmable Gate Arrays (FPGAs).

### THERAPEUTIC MODALITIES

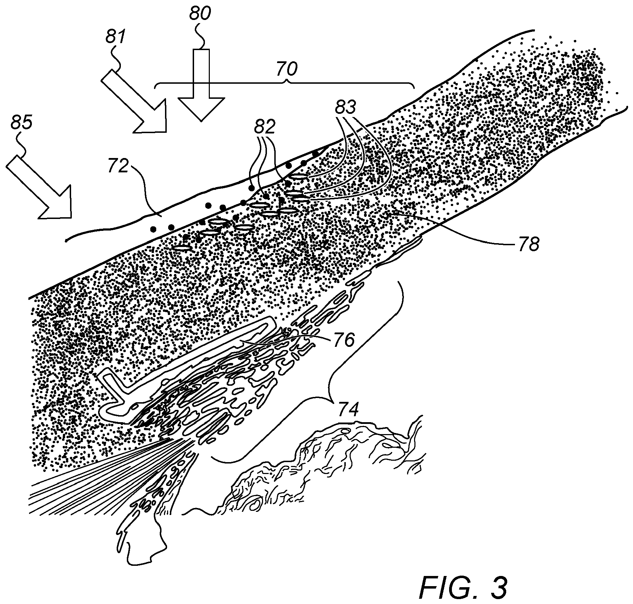

Fig. 3 is a schematic sectional illustration of structures in a limbal area 70 of an eye under treatment by laser radiation 80, in accordance with an embodiment of the invention. (The limbus itself falls roughly in the center of this area.) Limbal area 70 includes a corneal epithelium 72, containing epithelial stem cells 82. Laser radiation 80 is generated, for example, by optical unit 30 in system 20, as described above. The beam parameters of laser radiation 80, including the number of laser pulses, the energy and duration per pulse, and the focal spot size of the laser beam on epithelium 72, are chosen so as to stimulate stem cells 82 selectively.

Beneath the epithelium, the cornea contains a stroma 78, containing stromal stem cells 83, and an endothelium 74, including the trabecular meshwork and Schlemm's canal 76 (among other structures), as well as endothelial stem cells (not shown in the figure). In the present embodiment, the laser beam parameters are chosen so as to minimize penetration of laser energy into these inner layers and thus avoid stimulation of the stem cells in these layers. This selectivity may be enhanced, for example, by directing laser radiation 81 to impinge on the area of epithelial stems cells 82 in an oblique direction. (The term "oblique," as used in the present description and in the claims, means that the laser beam impinges on the eye at an angle of at least 5^{O} relative to the direction of the optical axis of the eye, i.e., an axis passing through the center of the pupil to the macula. In some embodiments, the laser beam may impinge on the eye at an angle of 10^{O} or more.)

In alternative embodiments, however, the laser beam parameters may be modified, for example by increasing the fluence and/or the focal spot size, in order to stimulate the stem cells in stroma 78, as well as in endothelium 74. Increasing the energy dosage sufficiently to affect the trabecular meshwork can also reduce intraocular pressure, as described in the above-mentioned U.S. Patent 10,702,416, in conjunction with the effect of the laser radiation on the endothelial stem cells. Additionally or alternatively, laser radiation 85 may be directed to impinge on the eye at the edge of limbal region 70, at an oblique angle selected so as to stimulate the endothelial stem cells, while avoiding the epithelial and stromal stem cells.

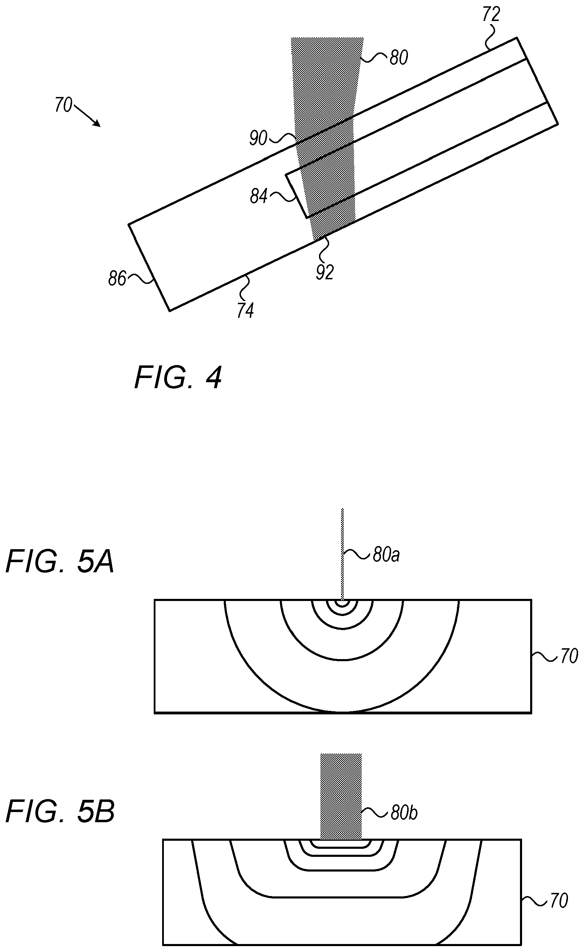

Fig. 4 is a schematic, idealized sectional illustration of limbal area 70 of an eye showing propagation of unscattered laser radiation 80 through the area, in accordance with an embodiment of the invention. Laser radiation 80 impinges on an entrance spot 90 at a target location on epithelium 72, at the anterior surface of the eye. As noted earlier, spot 90 is typically no greater than 1 mm in diameter, and may be smaller. The radiation passes through a cornea 84 of the eye and impinges on endothelium 74 at an exit spot 92.

The intensity of the laser radiation drops sharply, typically by more than 90%, between entrance spot 90 and exit spot 92, due mainly to scattering of the radiation within the limbal tissues. Due to these scattering properties, the stimulation of the epithelial stem cells can be as much as twenty times stronger (or even more, depending on the wavelength of light and the specific patient eye anatomy) than that of the endothelial stem cells. This differential effect can be enhanced still more by focusing radiation 80 sharply onto entrance spot 90.

Figs. 5A and 5B are schematic sectional illustrations of tissue in limbal area 70 of an eye, showing distributions of optical fluence under irradiation by laser beams 80a and 80b of different focal properties, in accordance with an embodiment of the invention. Beam 80a is sharply focused onto the anterior surface of the eye, whereas beam 80b has a much larger spot size. The contours in the figures illustrate the relative decrease in fluence as the beams propagate through the tissue. The larger spot size of beam 80b increases the relative fluence reaching the endothelium and thus enables greater stimulation of internal structures, such as the stromal and endothelial stem cells.

In some embodiments, optical unit 30 (Fig. 1) is operated to emit pulses having a pulse duration that is less than 1 µs and an energy that is less than 5 mJ per pulse. Alternatively, the pulses may be still shorter, for example less than 100 ns. In some cases, it is advantageous that the pulse durations be less than 10 ns or even 5 ns. Additionally or alternatively, the pulse energy may be smaller, for example less than 3 mJ. Typically, the laser radiation is focused to impinge on each of the treated locations with a fluence that is less than 0.4 J/cm². Controller 44 typically actuates laser 48 and controls scanner 50 to apply sequences of multiple pulses at each of the treated locations on the anterior surface of the eye. Typically, 200 pulses at each location provide sufficient therapeutic effect, and 100 pulses or less may be preferred in order to accelerate the treatment and reduce collateral effects. For these same reasons, the cumulative energy that is delivered by optical unit 30 to each such location is no greater than 100 mJ, and may be less than 70 mJ or even 40 mJ.

In other embodiments, optical unit 30 may apply pulses of lower peak power but higher total energy to the anterior surface of the eye. In such embodiments, laser 48 emits pulses having a pulse duration that is greater than 1 ms but less than 500 ms and an energy that is less than 100 mJ per pulse. Alternatively, the pulses may be shorter, for example less than 100 ms. In some cases, it is advantageous that the pulse durations be less than 50 ms, and possibly less than 10 ms or even 5 ms. Additionally or alternatively, the pulse energy may be smaller, for example less than 50 mJ or possibly less than 5 mJ. Typically, the laser radiation is focused to impinge on each of the treated locations with a fluence that is less than 30 J/cm². Controller 44 typically actuates laser 48 and controls scanner 50 to apply sequences of multiple pulses at each of the treated locations, typically no more than 2000 pulses at each location. Alternatively, as in the preceding embodiment, 500 pulses or less, or even as few as 200 pulses at each location, may be preferred in order to accelerate the treatment and reduce collateral effects. The cumulative energy that is delivered by optical unit 30 to each such location is no greater than 10 J, and may be less than 1 J or even 400 mJ.

### ALTERNATIVE EMBODIMENTS

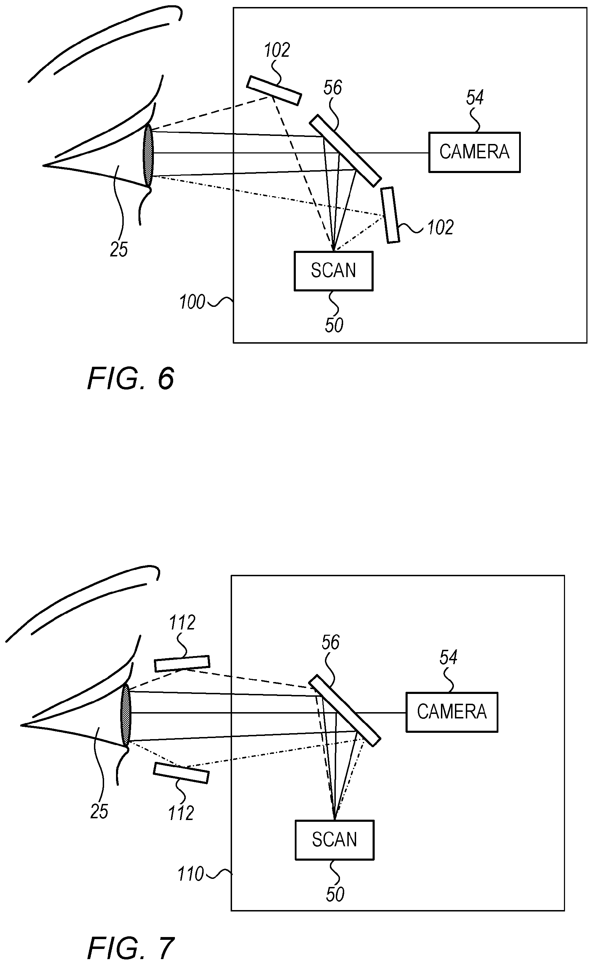

Figs. 6 and 7 are schematic sectional views of systems 100 and 110 for ophthalmic treatment, respectively, in accordance with alternative embodiments of the invention. These systems are similar to system 20, as shown in Figs. 1 and 2, but include additional optical components to facilitate directing the laser radiation to impinge on the anterior surface of the eye at an oblique angle relative to the direction of the optical axis of the eye. The laser radiation may be pulsed, as described above, or continuous wave (CW). As explained earlier, this sort of oblique-angle irradiation is useful in selectively stimulating the stem cells in a particular layer of the cornea, such as the epithelial, stromal, or endothelial stem cells.

In system 100, a ring mirror 102 is positioned around the optical axis of the eye, surrounding the optical axis and beam combiner 56. In one embodiment, ring mirror 102 comprises a unitary ellipsoidal reflector. Alternatively, ring mirror 102 may comprise multiple mirror segments, for example flat segments, which are arranged to emulate the optical performance of an ellipsoid. In any case, when scanner 50 directs the laser beam toward ring mirror 102, the beam will be reflected toward the eye at an oblique angle relative to the optical axis.

In system 110, a ring mirror 112 is attached externally to the optical unit, similarly surrounding the optical axis of the eye. Ring mirror 112 may be constructed and mounted, for example, in the manner in which a frustum-shaped conduit is attached to the optical unit in PCT Patent Application PCT/IB2020/052020, filed March 9, 2020, whose disclosure is incorporated herein by reference. In this case, too, when scanner 50 directs the laser beam toward ring mirror 112, the beam will be reflected toward the eye at an oblique angle relative to the optical axis.

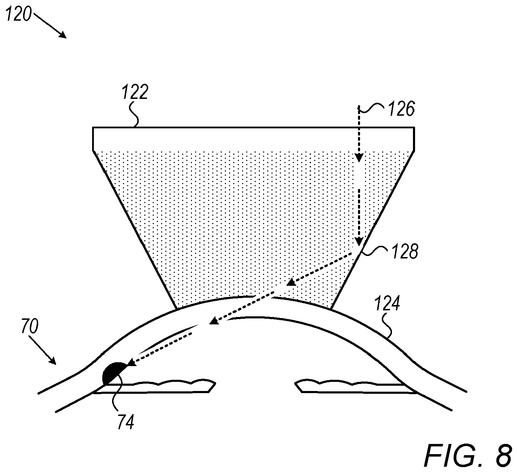

Fig. 8 is a schematic sectional illustration of apparatus 120 for ophthalmic treatment, in accordance with yet another embodiment of the invention. In this embodiment, a gonioscope 122 is held in contact with a cornea 124 of an eye. A laser beam 126 is directed to reflect from a side mirror surface 128 of gonioscope 122, and thus to pass obliquely through the transparent area of cornea 124 and impinge on endothelial tissue in the vicinity of trabecular meshwork 74. This sort of optical approach is used in some methods of laser trabeculoplasty that are known in the art.

In the present embodiment, however, the laser pulse energy is far lower than the threshold for effective trabeculoplasty: For example, laser beam 126 may comprise pulses with a pulse duration that is less than 1 µs and an energy that is less than 0.1 mJ per pulse, with a fluence no greater than 0.1 J/cm². Alternatively, the pulses may have a pulse duration between 1 and 500 ms and fluence no greater than 10 J/cm². These laser pulses will stimulate the endothelial stem cells. Due to the high angle of incidence of the laser beam and the high scattering loss of the tissue in limbal area 70, relatively little laser energy will penetrate outward from the corneal endothelium to the epithelial and stromal stem cells. In this case, the endothelial stem cells will be stimulated selectively.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

The following numbered clauses on pages 14 to 20 of the present description correspond to the claims of European patent application no. 20864109.2 as filed. The claims of the present application as filed, which is divided from European patent application no. 20864109.2, can be found on the subsequent pages 21 and 22 of the specification which begin with the heading "CLAIMS".
1. Apparatus for ophthalmic treatment, comprising:
   an optical unit, comprising:
      a camera, which is configured to capture an image of an eye of a patient;
      a laser, which is configured to emit pulses of laser radiation; and
      beam conditioning and scanning optics, which are configured to focus and direct the laser radiation to impinge on an anterior surface of the eye; and
   a controller, which is configured to analyze the image so as to identify a limbus of the eye, and to control the optical unit so that the laser radiation impinges on a set of one or more locations on the anterior surface of the eye that are in a vicinity of the limbus with a pulse duration that is less than 1 µs and an energy that is less than 5 mJ per pulse, whereby the laser radiation stimulates stem cells at the one or more locations.
2. The apparatus according to clause 1, wherein the laser radiation is focused to impinge on each of the one or more locations with a beam diameter no greater than 1 mm.
3. The apparatus according to clause 1, wherein the pulse duration is less than 100 ns.
4. The apparatus according to clause 3, wherein the pulse duration is less than 10 ns.
5. The apparatus according to clause 4, wherein the pulse duration is less than 5 ns.
6. The apparatus according to clause 1, wherein the energy per pulse is less than 3 mJ.
7. The apparatus according to clause 1, wherein the laser radiation is focused to impinge on each of the one or more locations with a fluence less than 0.4 J/cm².
8. The apparatus according to any of clauses 1-7, wherein the controller is configured to actuate the laser so as to apply multiple pulses to each of the one or more locations.
9. The apparatus according to clause 8, wherein the multiple pulses comprise no more than 200 pulses at each location.
10. The apparatus according to clause 9, wherein the multiple pulses comprise no more than 100 pulses at each location.
11. The apparatus according to clause 8, wherein the multiple pulses deliver the laser radiation with a cumulative energy that is no greater than 100 mJ at each location.
12. The apparatus according to clause 11, wherein the cumulative energy is no greater than 70 mJ.
13. The apparatus according to clause 12, wherein the cumulative energy is no greater than 40 mJ.
14. The apparatus according to any of clauses 1-7, wherein the one or more locations on the anterior surface of the eye are displaced by at least 1 mm from the limbus.
15. The apparatus according to clause 14, wherein the one or more locations on the anterior surface of the eye are displaced by at least 2 mm from the limbus.
16. The apparatus according to any of clauses 1-7, wherein the beam conditioning and scanning optics are configured to direct the laser radiation to impinge on the anterior surface of the eye at an oblique angle relative to a direction of an optical axis of the eye.
17. The apparatus according to clause 16, wherein the beam conditioning and scanning optics comprise a ring mirror, which is positioned around the optical axis of the eye so as to reflect the beam toward multiple locations in the vicinity of the limbus at oblique angles.
18. Apparatus for ophthalmic treatment, comprising:
   an optical unit, comprising:
      a camera, which is configured to capture an image of an eye of a patient;
      a laser, which is configured to emit pulses of laser radiation; and
      beam conditioning and scanning optics, which are configured to focus and direct the laser radiation to impinge on an anterior surface of the eye; and
   a controller, which is configured to analyze the image so as to identify a limbus of the eye, and to control the optical unit so that the laser radiation impinges on a set of one or more locations on the anterior surface of the eye in a vicinity of the limbus with a pulse duration that is between 1 ms and 500 ms and an energy that is less than 100 mJ per pulse, whereby the laser radiation stimulates stem cells at the one or more locations.
19. The apparatus according to clause 18, wherein the laser radiation is focused to impinge on each of the one or more locations with a beam diameter no greater than 1 mm.
20. The apparatus according to clause 18, wherein the pulse duration is less than 50 ms.
21. The apparatus according to clause 20, wherein the pulse duration is less than 10 ms.
22. The apparatus according to clause 21, wherein the pulse duration is less than 5 ms.
23. The apparatus according to clause 18, wherein the energy per pulse is less than 50 mJ.
24. The apparatus according to clause 20, wherein the energy per pulse is less than 5 mJ.
25. The apparatus according to clause 18, wherein the laser radiation is focused to impinge on each of the one or more locations with a fluence less than 30 J/cm².
26. The apparatus according to any of clauses 18-25, wherein the controller is configured to actuate the laser so as to apply multiple pulses to each of the one or more locations.
27. The apparatus according to clause 26, wherein the multiple pulses comprise no more than 2000 pulses at each location.
28. The apparatus according to clause 27, wherein the multiple pulses comprise no more than 500 pulses at each location.
29. The apparatus according to clause 28, wherein the multiple pulses comprise no more than 200 pulses at each location.
30. The apparatus according to clause 26, wherein the multiple pulses deliver the laser radiation with a cumulative energy that is no greater than 10 J at each location.
31. The apparatus according to clause 30, wherein the cumulative energy is no greater than 1 J.
32. The apparatus according to clause 31, wherein the cumulative energy is no greater than 400 mJ.
33. The apparatus according to any of clauses 18-25, wherein the one or more locations on the anterior surface of the eye are displaced by at least 1 mm from the limbus.
34. The apparatus according to clause 33, wherein the one or more locations on the anterior surface of the eye are displaced by at least 2 mm from the limbus.
35. The apparatus according to any of clauses 18-25, wherein the beam conditioning and scanning optics are configured to direct the laser radiation to impinge on the anterior surface of the eye at an oblique angle relative to a direction of an optical axis of the eye.
36. The apparatus according to clause 35, wherein the beam conditioning and scanning optics comprise a ring mirror, which is positioned around the optical axis of the eye so as to reflect the beam toward multiple locations in the vicinity of the limbus at oblique angles.
37. A method for ophthalmic treatment, comprising:
   identifying a limbus of an eye of a patient; and
   directing pulses of laser radiation to impinge on a set of one or more locations on an anterior surface of the eye in a vicinity of the limbus with a pulse duration that is less than 1 µs and an energy that is less than 5 mJ per pulse, so as to stimulate stem cells at the one or more locations.
38. The method according to clause 37, wherein directing the pulses of laser radiation comprises focusing the laser radiation to impinge on each of the one or more locations with a beam diameter no greater than 1 mm.
39. The method according to clause 37, wherein the pulse duration is less than 100 ns.
40. The method according to clause 39, wherein the pulse duration is less than 10 ns.
41. The method according to clause 40, wherein the pulse duration is less than 5 ns.
42. The method according to clause 37, wherein the energy per pulse is less than 3 mJ.
43. The method according to clause 37, wherein directing the pulses comprises focusing the laser radiation to impinge on each of the one or more locations with a fluence less than 0.4 J/cm².
44. The method according to any of clauses 37-43, wherein directing the pulses of laser radiation comprises applying multiple pulses to each of the one or more locations.
45. The method according to clause 44, wherein the multiple pulses comprise no more than 200 pulses at each location.
46. The method according to clause 45, wherein the multiple pulses comprise no more than 100 pulses at each location.
47. The method according to clause 44, wherein applying the multiple pulses comprises delivering the laser radiation with a cumulative energy that is no greater than 100 mJ at each location.
48. The method according to clause 47, wherein the cumulative energy is no greater than 70 mJ.
49. The method according to clause 48, wherein the cumulative energy is no greater than 40 mJ.
50. The method according to any of clauses 37-43, wherein the one or more locations on the anterior surface of the eye are displaced by at least 1 mm from the limbus.
51. The method according to clause 50, wherein the one or more locations on the anterior surface of the eye are displaced by at least 3 mm from the limbus.
52. The method according to any of clauses 37-43, wherein directing the pulses comprises directing the laser radiation to impinge on the anterior surface of the eye at an oblique angle relative to a direction of an optical axis of the eye.
53. The method according to clause 52, wherein directing the laser radiation comprises positioning a ring mirror around the optical axis of the eye so as to reflect the beam toward multiple locations in the vicinity of the limbus at oblique angles.
54. A method for ophthalmic treatment, comprising:
   identifying a limbus of an eye of a patient; and
   directing pulses of laser radiation to impinge on a set of one or more locations on an anterior surface of the eye in a vicinity of the limbus with a pulse duration that is less than 100 ms and an energy that is less than 100 mJ per pulse, so as to stimulate stem cells at the one or more locations.
55. The method according to clause 54, wherein directing the pulses of laser radiation comprises focusing the laser radiation to impinge on each of the one or more locations with a beam diameter no greater than 1 mm.
56. The method according to clause 54, wherein the pulse duration is less than 50 ms.
57. The method according to clause 56, wherein the pulse duration is less than 10 ms.
58. The method according to clause 57, wherein the pulse duration is less than 5 ms.
59. The method according to clause 54, wherein the energy per pulse is less than 50 mJ.
60. The method according to clause 59, wherein the energy per pulse is less than 5 mJ.
61. The method according to clause 54, wherein directing the pulses comprises focusing the laser radiation to impinge on each of the one or more locations with a fluence less than 30 J/cm².
62. The method according to any of clauses 54-61, wherein directing the pulses of laser radiation comprises applying multiple pulses to each of the one or more locations.
63. The method according to clause 62, wherein the multiple pulses comprise no more than 2000 pulses at each location.
64. The method according to clause 63, wherein the multiple pulses comprise no more than 500 pulses at each location.
65. The method according to clause 64, wherein the multiple pulses comprise no more than 200 pulses at each location.
66. The method according to clause 62, wherein applying the multiple pulses comprises delivering the laser radiation with a cumulative energy that is no greater than 10 J at each location.
67. The method according to clause 66, wherein the cumulative energy is no greater than 1 J.
68. The method according to clause 67, wherein the cumulative energy is no greater than 400 mJ.
69. The method according to any of clauses 54-61, wherein the one or more locations on the anterior surface of the eye are displaced by at least 1 mm from the limbus.
70. The method according to clause 69, wherein the one or more locations on the anterior surface of the eye are displaced by at least 3 mm from the limbus.
71. The method according to any of clauses 54-61, wherein directing the pulses comprises directing the laser radiation to impinge on the anterior surface of the eye at an oblique angle relative to a direction of an optical axis of the eye.
72. The method according to clause 71, wherein directing the laser radiation comprises positioning a ring mirror around the optical axis of the eye so as to reflect the beam toward multiple locations in the vicinity of the limbus at oblique angles.
73. Apparatus for treating an eye of a patient, the apparatus comprising:
   an optical unit, comprising:
      a laser, which is configured to emit laser radiation;
      one or more mirrors positioned around an optical axis of the eye; and
      beam conditioning and scanning optics, which are configured to focus and direct the laser radiation to reflect from the one or more mirrors so as to impinge on an anterior surface of the eye at an oblique angle relative to the optical axis; and
   a controller, which is configured to control the optical unit so that the laser radiation impinges on the eye at the oblique angle in a set of one or more locations in a vicinity of the limbus with a fluence selected so as to stimulate stem cells at the one or more locations.
74. The apparatus according to clause 73, wherein the one or more mirrors comprise a ring mirror, which surrounds the optical axis.
75. The apparatus according to clause 73 or 74, wherein the controller is configured to control the optical unit so that the laser radiation impinges on and stimulates the stem cells in a epithelium of the eye.
76. The apparatus according to clause 73 or 74, wherein the controller is configured to control the optical unit so that the laser radiation impinges on and stimulates the stem cells in a stroma of the eye.
77. The apparatus according to clause 73 or 74, wherein the controller is configured to control the optical unit so that the laser radiation impinges on and stimulates the stem cells in an endothelium of the eye.
78. A method for ophthalmic treatment, comprising:
   placing a gonioscope in contact with a cornea of an eye of a patient; and
   directing pulses of laser radiation through the gonioscope to reflect from a side mirror surface of the gonioscope and then to pass obliquely through the cornea and impinge on endothelial tissue in a vicinity of a trabecular meshwork of the eye with a pulse energy and duration selected so as to stimulate stem cells in the endothelial tissue.
79. The method according to clause 78, wherein the pulses have a pulse duration that is less than 1 µs and an energy that is less than 0.1 mJ per pulse.
80. The method according to clause 79, wherein directing the pulses comprises controlling the laser radiation to impinge on the endothelial tissue with a fluence no greater than 0.1 J/cm².
81. The method according to any of clauses 78-80, wherein the pulses have a pulse duration that is between 1 and 500 ms and a fluence no greater than 10 J/cm².

## Claims

1. Apparatus (20) for ophthalmic treatment, comprising:
an optical unit (30), comprising:
a camera (54), which is configured to capture an image of an eye (25) of a patient;
a laser (48), which is configured to emit pulses of laser radiation; and
beam conditioning and scanning optics (49, 50), which are configured to focus and direct the laser radiation to impinge on an anterior surface of the eye; and
a controller (44), which is configured to analyze the image so as to identify a limbus of the eye, and to control the optical unit so that the laser radiation impinges on a set of one or more locations on the anterior surface of the eye in a vicinity of the limbus with a pulse duration that is between 1 ms and 500 ms and an energy that is less than 100 mJ per pulse, whereby the laser radiation stimulates stem cells at the one or more locations.

2. The apparatus according to claim 1, wherein the laser radiation is focused to impinge on each of the one or more locations with a beam diameter no greater than 1 mm.

3. The apparatus according to claim 1, wherein the pulse duration is less than 50 ms.

4. The apparatus according to claim 3, wherein the pulse duration is less than 10 ms.

5. The apparatus according to claim 1, wherein the energy per pulse is less than 50 mJ.

6. The apparatus according to claim 5, wherein the energy per pulse is less than 5 mJ.

7. The apparatus according to claim 1, wherein the laser radiation is focused to impinge on each of the one or more locations with a fluence less than 30 J/cm².

8. The apparatus according to any of claims 1-7, wherein the controller is configured to actuate the laser so as to apply multiple pulses to each of the one or more locations.

9. The apparatus according to claim 8, wherein the multiple pulses comprise no more than 2000 pulses at each location.

10. The apparatus according to claim 8, wherein the multiple pulses deliver the laser radiation with a cumulative energy that is no greater than 10 J at each location.

11. The apparatus according to claim 10, wherein the cumulative energy is no greater than 1 J.

12. The apparatus according to any of claims 1-7, wherein the one or more locations on the anterior surface of the eye are displaced by at least 1 mm from the limbus.

13. The apparatus according to claim 12, wherein the one or more locations on the anterior surface of the eye are displaced by at least 2 mm from the limbus.

14. The apparatus according to any of claims 1-7, wherein the beam conditioning and scanning optics are configured to direct the laser radiation to impinge on the anterior surface of the eye at an oblique angle relative to a direction of an optical axis of the eye.

15. The apparatus according to claim 14, wherein the beam conditioning and scanning optics comprise a ring mirror, which is positioned around the optical axis of the eye so as to reflect the beam toward multiple locations in the vicinity of the limbus at oblique angles.
